(19) Europäisches Patentamt
European Patent Office
Office européen des brevets

(11) **EP 3 087 960 B1**

(12) **EUROPEAN PATENT SPECIFICATION**

(45) Date of publication and mention
of the grant of the patent:
**19.12.2018 Bulletin 2018/51**

(51) Int Cl.:
*A61F 13/00* (2006.01)     *A61F 13/02* (2006.01)

(21) Application number: **14874202.6**

(22) Date of filing: **23.10.2014**

(86) International application number:
**PCT/CN2014/089318**

(87) International publication number:
**WO 2015/096543 (02.07.2015 Gazette 2015/26)**

(54) **WOUND DRESSING CONTAINING THREE-LAYER FABRIC AND WOUND DRESSING MANUFACTURING METHOD**

WUNDVERBAND MIT DREISCHICHTIGEM GEWEBE UND WUNDVERBANDHERSTELLUNGSVERFAHREN

PANSEMENT CONTENANT UN TISSU À TROIS COUCHES ET PROCÉDÉ DE FABRICATION D'UN PANSEMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.12.2013 CN 201310728101**

(43) Date of publication of application:
**02.11.2016 Bulletin 2016/44**

(73) Proprietor: **Foshan United Medical Technologies Ltd**
**Guangdong 528225 (CN)**

(72) Inventors:
• **WANG, Xiaodong**
  **Guangdong 528225 (CN)**
• **MO, Xiaohui**
  **Guangdong 528225 (CN)**
• **FENG, Caixia**
  **Guangdong 528225 (CN)**
• **XIAO, Jianpeng**
  **Guangdong 528225 (CN)**

(74) Representative: **Wilson Gunn**
**Charles House**
**148/9 Great Charles Street**
**Birmingham B3 3HT (GB)**

(56) References cited:
WO-A1-00/41661          CN-A- 102 076 291
CN-A- 102 525 737       CN-A- 103 126 806
CN-A- 103 735 359       GB-A- 2 489 947
US-A- 5 632 731         US-A- 5 797 894
US-A1- 2007 238 382     US-A1- 2009 221 946
US-A1- 2011 280 926

## Description

### Technical field

This invention relates to a three-layered wound dressing and its method of manufacturing.

### Background art

Advanced wound dressings, such as fibrous wound dressings are used regularly in the current practice for the management of chronic wounds. Typical dressings are alginate, chitosan, carboxymethyl cellulose (CMC) etc. The main features of these dressings are absorption and gelling which also help to retain the moisture after absorption providing an optimum environment for wound healing.

In addition to the single component wound dressing, there are some dressings made from many different fibres blended and combined together, such as blending non-gelling fibres with gelling fibres to provide a dressing with a good absorbency and a reasonable wet strength.

EP0740554 and US6471982 disclosed a dressing made from gelling fibre such as alginate blended with non-gelling fibres such as cellulose fibres.

US6458460 and EP0927013 disclosed another wound dressing which is made by blending two gelling fibres. One of them is carboxymethyl cellulose and the other is alginate. The dressing is believed to be more absorbent for wound exudates. US7385101 disclosed a wound dressing that is made by blending silver coated nylon fibres and absorbent fibres. The dressing contains antimicrobial agents and can be used to treat the infected wounds.

The above mentioned dressings have some advantage of combined absorbency, wet strength and antimicrobial property. But the dressings have some limitations due to the nature of blending which is an even mix of two or more types of fibres. Typical weaknesses are uniform density and uniform distribution of two materials, limiting the dressing's ability to provide different functions to different situations. Also due to the loose structure of the dressing surface, some fibres are inevitably left on the wound bed, making them difficult to remove. Sometimes irrigation method must be used to clean these fibres, causing more work for health care providers, and more pain to patients.

However there are some other ways to make the dressing with certain specific functions by multi-layer structured dressing, in addition to fibre blending technique. EP0575090B1 provided a dressing to absorb wound exudates by containing alginic acid housed in perforated bags interconnected by a string. Wound exudates can pass the perforation of the bags and be absorbed by the alginic acid.

EP0788378B1 disclosed a three layered wound dressing. The first layer of the dressing is some absorbent material such as alginate, chitosan or their derivatives which can help wound healing. The second layer is a fibrous fabric which is more hydrophilic than the first layer. The third layer is a material with high moisture vapor transport rate (MVTR) such as polyurethane (PU) film. This design may also leave the fibre residuals onto the wound bed.

WO2010/109239A1 and CN102481208A provided a layered dressings which also had three layers. The first layer is a gelling material such as hydrocolloid or hydrogel; the second layer has a higher hardness such as polyester; the third layer is a soft material with low surface friction. The dressing can absorb and retain wound exudates, and provide a moist environment. This design uses hydrocolloid as the first layer, therefore does not have the softness and conformity of the fibrous dressing.

US2011/0280926A1 and CN102076291A disclosed a multi-layered wound dressing. The dressing contains a wound contact layer and an absorption layer. The absorption layer uses hydrophilic PU foam. Due to the employment of polyurethane material, the dressing's conformity is not as good as that of the fibrous dressing.

WO2007/117237A1 and CN101460202A disclosed an absorbable multilayer haemostatic material. The dressing contains an absorbable nonwoven fabric and an absorbable woven or knitted fabric of clotting enzyme and/or fibrous protein. The product however does not have a good absorption property required for the management of heavily exudating wounds.

US6552244 and EP1139946 disclosed another multi-layered wound dressing. The top layer is an absorbent layer having high absorbency; and the middle layer is a transmission layer having a high moisture vapor transmission rate overlying the side of the absorbent layer. The other layer is a spreading layer. This dressing can absorb the wound exudates and transmit the absorbed fluid to the spreading layer. The design however has limited ability to provide a moist environment.

CN103126806A describes the manufacture of a wound dressing comprising a three layers structure in which the outer layers are made of sol fibres and the middle layer is made of woven fabric, knitted fabric or other non-woven fabric. The specification is silent on the weights and densities of the outer layers, and in relation to the middle layer, describes fabric having a linear fibre density of between 100 dtex and 2000 dtex.

It is obvious that all above dressings have some limitations in the management of chronic wounds such as conformity, softness and moisture retention. It is therefore the objectives of the present invention to address all these problems.

### Disclosure of invention

The objective of the present invention is to find a wound dressing and its method of manufacturing that can provide absorbency, fluid retention and moisture management.

The first element of the present invention provides a method of manufacturing a wound dressing, the wound dressing comprising: a first outer layer, a second outer layer, and a middle layer disposed between the first outer layer and the second outer layer; wherein the volume density of the first outer layer and a volume density of the second outer layer are greater than that of the middle layer;, wherein the method comprises the steps of: a) providing first and second outer layers, wherein the weights of the first outer layer and the second outer layer are between 8 and 140 grams per square meter (gsm), and the volume densities of the first outer layer and the second outer layer are between 0.08 and 0.32 g/cm3; b) providing a middle layer comprising at least one of the following fibres: alginate, chitosan, cellulose, carboxymethyl chitosan, acylated chitosan, carboxymethyl cellulose, carboxyethyl cellulose, water insoluble cellulose alkyl sulfonate fibre, bi-component fibre, polyvinyl alcohol, polypropylene, polyester, polyamide, polyacrylonitrile, cross-linked acrylates copolymer super absorbent fibres, and wood pulp and the weight of the middle layer is between 60-600 gsm; the fibre linear density of the middle layer is between 0.5-10 dtex; and the fibre length thereof is between 3-150 mm; and c) bonding the layers together. Either the first layer or the second layer or both is a fabric containing alginate, or chitosan, or cellulose, or carboxymethyl chitosan, or acylated chitosan, or carboxymethyl cellulose (CMC), or carboxy ethyl cellulose (CEC), or water insoluble cellulose alkyl sulfonate fibre, or polyvinyl alcohol fibre (PVA), or polypropylene (PP), or polyester (PET), or Polyamide (PA), or Polyacrylonitrile (PAN).

Either the first layer or the second layer or both may contain antimicrobial agent at the concentration of 0.01-25% by weight of the first layer or the second layer, preferably 0.1-20%. The middle layer can also contain antimicrobial agent at the concentration of 0.01-20% by weight of the third (middle) layer, preferably 01-10%. The preferred antimicrobial agent is silver.

Either the first layer or the second layer or both is a knitted fabric, or woven fabric or a nonwoven fabric. The third (middle) layer may be a nonwoven fabric. The said knitted or woven fabrics are made from staple yarns or filament. The linear density of the said fibre or the said filament is between 0.5-10 dtex, preferably 1-6 dtex.

The third (middle) can contain a blend of two or more of the following fibres: alginate, chitosan, cellulose, carboxymethyl chitosan, acylated chitosan, carboxymethyl cellulose (CMC), carboxy ethyl cellulose (CEC), water insoluble cellulose alkyl sulfonate fibre, bi-component fibre, polyvinyl alcohol fibre (PVA), polypropylene (PP), polyester (PET), Polyamide (PA), Polyacrylonitrile (PAN), cross-linked acrylates copolymer super absorbent fibres (SAF), wood pulp. The weight of the middle layer is preferably between 80-500 gsm, most preferably 100-400 gsm.

The fibre linear density of the middle layer is

preferably between 1-6 dtex. The fibre length of the middle layer is preferably between 5-75 mm.

The nonwoven of these layers can be made by needle punching, hydroentanglement, thermal bonding and chemical bonding.

The above layers can be laminated together by needle punching, or thermal binding, or chemical (adhesive) bonding or ultrasonic welding, or stitch bonding. If the thermal bonding or ultrasonic welding or stitch bonding method is used, one or more patterns can be created during the lamination, such as square, triangle, rectangle, diamond, circle or dot.

The method of the present invention also provides a three-layered fabric for use in the management of chronic wounds or the moisture management.

The method may include A) providing fibres for the three layers, and taking measurements of the fibre parameters; B) manufacturing the web for the middle layer; C) converting the web into nonwoven for the third layer; D) laminating the third layer with the first and the second layers; E) processing the laminated fabric such as by cutting, packing and sterilizing into the three-layered wound dressings.

The benefits of the present invention are:

1. The fabric of the wound dressing produced by the methods of the present invention contains three layers, i.e. the first layer, the second layer and the third (middle) layer. The middle layer has a smaller volume density than the two outer layers, so it is easier to absorb and retain moisture or wound exudates.

2. The first layer and the second layer have higher density than the middle layer, so that any moisture or fluid that has been absorbed or contained in the middle layer is locked between the two outer layers, preventing the absorbed moisture or exudates from coming out thus preventing possible body infection or wound maceration. This will make the outer layers still feel dry after absorption.

3. A dressing with such a three-layered structure has a higher wet strength so it can be removed as one piece without any loose fibre residual left behind. There is less free fibre on the surface of the outer layers.

## Drawings

Fig.1:    The effect of the zone of inhibition of the sample from example 2 against Staphylococcus aureus at 1 day

Fig.2:    The effect of the zone of inhibition of the sample from example 5 against Pseudomonas aeruginosa at 1 day

Fig.3:    The structure of the three-layered wound dressing.

Fig.4:    The flow chart of the manufacturing method

The present invention can be illustrated by the following methods or descriptions, in order to fully understand the method and the technology. The scope of the invention is not limited by the after mentioned examples or descriptions.

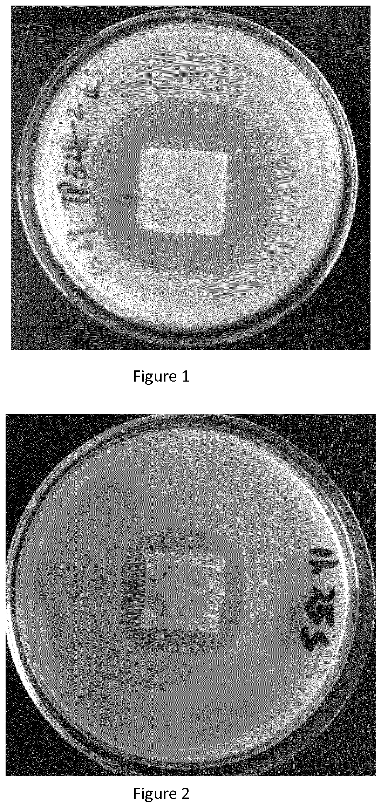

The method of the manufacturing of the present invention can be described as follow, together with Fig 1- Fig 4.

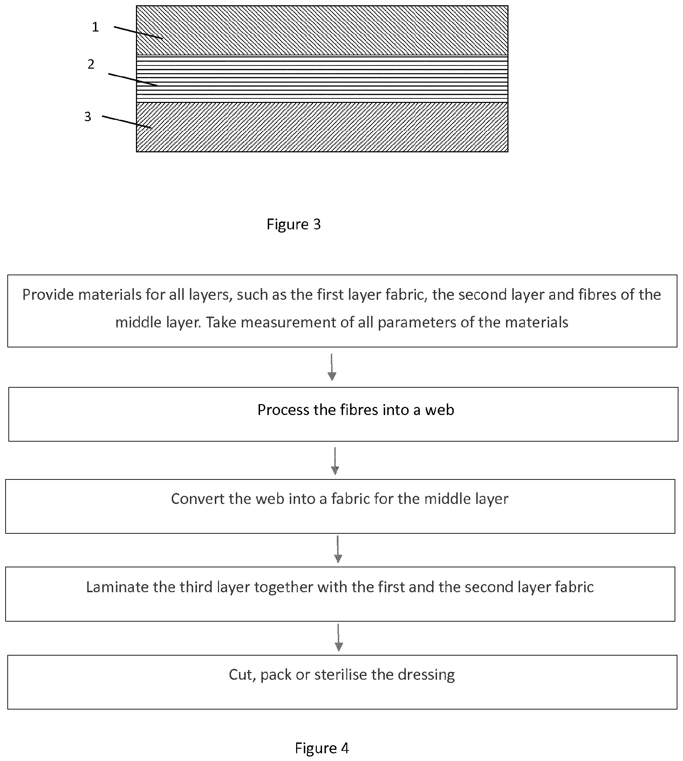

The present invention provides a three-layered fabric, as shown in Fig 3, in which 1 is the first layer, 3 is the second layer, 2 is the middle (third) layer. The first and the second layers are the outer layers of the dressing, the third layer is positioned between the first and the second layers. The volume densities of the first layer and the second layer are greater than that of the middle layer. The three layers are bonded together. The weight of the first layer and the second layer are between 8-140 gsm, preferably 10-100 gsm. The densities of the first layer and the second layer are between 0.08-0.32 g/cm3, preferably 0.08-0.25 g/cm3. The wound dressing made from this fabric has an absorbency of greater than 10 g/100cm2, and has a sufficient wet strength to allow an intact removal from the wound bed after absorption.

The first layer and/or the second layer can be a fabric of alginate, or chitosan, or cellulose, or chemically modified chitosan, chemically modified cellulose, or PVA, or PP, or PET, or PA or PAN. It can also be made by blending two or more materials from the above mentioned fibres. The first layer and the second layer can be the same fabric or two different fabrics. This means that the fabric can be symmetric or asymmetric.

The alginate can be a high M alginate, or a high G alginate. It can also be a calcium or calcium/sodium alginate. The chitosan can be a chitosan with a degree of deacetylation of 80% or above. The chemically modified chitosan can be carboxymethyl chitosan or acylated chitosan. The chemically modified chitosan has an absorbency of 500% or above. The cellulose fibre can be traditional viscose fibre or solvent spun cellulose fibre such as lyocell. The chemically modified cellulose can be carboxymethyl cellulose or carboxy ethyl cellulose, or water insoluble cellulose alkyl sulfonate fibre. The absorbency of the chemically modified fibre is 500% or above. The first and/or the second layer can have an antimicrobial agent such as silver, PHMB, or honey. The preferred agent is silver, such as silver compound or silver complex. Silver can be applied to the fibre first then made into fabric, or it can be applied directly to the fabric. The silver content can be 0.01-25% by weight of the respective fabric layer, preferably 0.1-20%.

It is not the intention of the present invention to detail the method of manufacturing silverfibre/fabric, the following methods can be considered forthe application of silver to the fibre or fabric:

1. Add antimicrobial agent such as silver nitrate, silver chloride, silver carbonate, or silver sodium zirconium hydrogen phosphate into the fibre spinning polymer solution (also referred to as dope), then extrude the solution into fibres to make the silver fibres;
2. Spray the silver containing solution to the fibre or fabric surface, such as spray nano silver solution to fibre or fabric surface;
3. Coat silver onto the surface of fibre or fabric.

Either the first layer or the second layer or both can be woven fabric or knitted fabric or nonwoven fabric. The woven fabric is manufactured with a weaving process and equipment. The knitted fabric is manufactured with a knitting process and equipment. The nonwoven fabric is manufactured with a carding and nonwoven process and equipment.

For the woven and knitted fabric, it can be made with staple fibre yarns or filaments, the fibre or filament linear density can be between 0.5-10 dtex, preferably 1-6 dtex. For nonwoven fabric, it can be made with needle punching method, or hydroentanglement method, or thermal bonding method, or chemical bonding method. The hydroentanglement method can only be applied to the non-gelling fibres. The thermal bonding method can only be applied to fibres manufactured from the hot melt method.

The middle layer can contain alginate fibre, chitosan fibre, viscose fibres, lyocell fibres, carboxymethyl chitosan fibres, acylated chitosan fibres, carboxymethyl cellulose fibres, carboxy ethyl cellulose fibres, water insoluble cellulose alkyl sulfonate fibres, bi-component fibres. It can also contain PA fibres, PP fibres, PET fibres, PA fibres, PAN fibres, cross-linked acrylate copolymer fibres, wood pulp fibres.

The middle layer can also be made by blending two or more following fibres: alginate fibre, chitosan fibre, viscose fibres, lyocell fibres, carboxymethyl chitosan fibres, acylated chitosan fibres, carboxymethyl cellulose fibres, carboxy ethyl cellulose fibres, water insoluble cellulose alkyl sulfonate fibres, bi-component fibres, PA fibres, PP fibres, PET fibres, PA fibres, PAN fibres, cross-linked acrylate copolymer fibres and wood pulp fibres. For example, by blending alginate and chitosan fibre, it will make the middle layer absorbent and hemostatic. To increase the absorbency, the fabric can be made by bending alginate and CMC fibres. For improving wet strength, the chemically modified fibres can be mixed with cellulose fibre such as lyocell.

The middle layer can also contain antimicrobial agents such as silver, PHMB or honey, preferably silver. The concentration of the antimicrobial agent is 0.01-20%, preferably 0.1-10%.

The weight of the middle layer fabric is 60-600 gsm, preferably 80-500 gsm, most preferably 100-400 gsm.

Staple fibres are used to manufacture the middle layer. The fibre length is between 3-150 mm, preferably 5-75 mm. The linear density of the fibre is 0.5-10 dtex, preferably 1-6 dtex. The middle layer is a nonwoven fabric manufactured by needle punching method, or thermal bonding method or chemical bonding method.

The 3 layers of the wound dressing produced by the methods of present invention can be laminated together by needle punching, or thermal bonding or ultrasonic welding or chemical (adhesive) bonding or stitch bonding. The needle punching and the chemical bonding are easier to use and can be used for all fibres. Thermal bonding and ultrasonic welding can only be used on fabric which contains hot melt spun fibres. The fabric made from the needle punching method feels soft and the chemically bonded fabric feels rigid. Thermally bonded fabric, the stitch bonded fabric and the fabric made with ultrasonic method can produce some pattern at the bonding points such as square, triangle, rectangle, diamond, circle or dot. These patterns may be used to prevent the lateral spreading of fluid, providing a lateral moisture locking function to the fabric.

The method of manufacturing a wound dressing according to the present invention can comprise the following steps:

A) Provide all raw materials i.e. all fibres or filaments for the three layers, and take measurement of all the parameters of the material;
B) Process the fibres into a web
C) Convert the web into a fabric for the middle layer;
D) Laminate the third layer together with the first and the second layer fabric;
E) Cut, pack or sterilise the dressing.

It is also important to describe the measurement of few fabric parameters:

1) Fabric weight
Cut the fabric into square or rectangle, measure the weight G (gram), then place the fabric onto a flat surface to measure its length L (cm) and the width W (cm). The fabric weight can be calculated as follows:

$$Gsm = G \times 100 \times 100 / (L \times W).$$

2) Fabric thickness or volume density
Place the fabric between the measuring feet of the thickness gauge, the diameter of the measurement feet is 30 mm, pressure 0.5 N. Take the reading of the fabric D (mm) to the nearest 0.001 mm.
Use the thickness and the data in 1) to calculate the volume density P (g/cm3) as follow:

$$P = G \times 10 / (L \times W \times D).$$

3) Fibre linear density: Follow the method provided in GB/T 14335 Linear density test method for man-made fibres
4) Fibre length: Follow the method provided in GB/T 14335 Linear density test method for man-made fibres

The following examples describe the manufacturing method and the structure of the fabric/dressing.

The solution A referred to in this application is a solution provided in BS EN 13726-2002 which contain 8.298g/l of sodium chloride and 0.368 g/l of calcium chloride dehydrate and distilled water.

**Example 1**

The first and the second fabric inf this example is PP fabric, the middle layer is Mid M alginate fabric.

The detailed manufacturing steps are:

A) The first and second layer use spun bond PP nonwoven, weight 12 gsm, volume density 0.08 g/cm3, white in colour.
The middle layer contains in-house made MG alginate fibres, linear density 3.0 dtex, fibre length 75 mm, the fibre's absorbency to Solution A is 1400%;
B) Take 500 g of alginate fibres, open and feed the fibres into a carding machine to make into a fibrous web;
C) Process the web into a needle punched felt as the middle layer fabric, needling density 60/cm2, fabric weight 99 gsm, volume density 0.07 gsm;
D) Place the above alginate felt between the first and the second fabrics, then feed them together into 2 needle machines respectively. The first machine needles downwards, the second upwards, needling density 100/cm2. This made the laminated three-layered fabric;
E) Cut the above laminated fabric into 10x10 cm, then pack and sterilize at a dosage of 25-50 KGy;

Test the absorbency of the finished dressing using the YY/T 047.1-2004 and EN 13726-1:2002/AC:2003 Test Method for Primary Wound Dressing. The results were 19 g/100cm2.

**Example 2**

The first and the second fabric in this example is PP fabric, the middle layer is Silver CMC fabric (silver carboxymethyl cellulose).

The detailed manufacturing steps are:

A) The first and second layer use spun bond PP nonwoven, weight 12 gsm, volume density 0.08 g/cm3, white in colour.
The middle layer contains in-house made silver carboxymethyl cellulose fibres, linear density 1.7 dtex, fibre length 50 mm, silver content 1%, the fibre's absorbency to Solution A is 1500%;
B) Take 500 g of silver CMC fibres, open and feed the fibres into a carding machine to make into a fibrous web;

C) Process the web into a needle punched felt as the middle layer fabric, needling density 60/cm2, fabric weight 130 gsm, volume density 0.078 gsm;

D) Place the above silver CMC felt between the first and the second fabrics, then feed them together into 2 needle machines respectively. The first machine needles downwards, the second upwards, needling density 100/cm2. This made the laminated three-layered fabric;

E) Cut the above laminated fabric into 10x10 cm, then pack and sterilize at a dosage of 25-50 KGy;

Test the absorbency of the finished dressing using the YY/T 047.1-2004 and EN 13726-1:2002/AC:2003 Test Method for Primary Wound Dressing. The results were 22 g/100cm2.

In order to assess the dressing's antimicrobial function, the samples from the above example was cut into a 2x2 cm piece, then placed onto a Petri-dish that has been covered with Staphylococcus aureus. The Petri-dish was then placed into an oven at a temperature of 37 °C for 24 hrs, and the growth of the bacteria around the dressing sample on the plate was observed (zone of inhibition). This demonstrates the dressing's ability to kill bacteria after 24 hrs contact time.

## Example 3

The first and the second fabrics of this example is hydroentanglement Lyocell fabric, the middle layer is a chemically modified chitosan fabric.

The detailed manufacturing steps are:

A) The first and second layer use hydroentanglement Lyocell nonwoven fabric, weight 30 gsm, volume density 0.13 g/cm3, white in colour.

The middle layer contains in-house made acylated chitosan fibres, linear density 2.0 dtex, fibre length 50 mm, the fibre's absorbency to Solution A is 2000%;

B) Take 500 g of acylated chitosan fibres, open and feed the fibres into a carding machine to make into a fibrous web;

C) Process the web into a needle punched felt as the middle layer fabric, needling density 40/cm2, fabric weight 125 gsm, volume density 0.075 gsm;

D) Place the above chitosan felt between the first and the second fabrics, then feed them together into 2 needle machines respectively. The first machine needles downwards, the second upwards, needling density 100/cm2. This made the laminated three-layered fabric;

E) Cut the above laminated fabric into 10x10 cm, then pack and sterilize at a dosage of 25-50 KGy;

Test the absorbency of the finished dressing using the YY/T 047.1-2004 and EN 13726-1:2002/AC:2003 Test Method for Primary Wound Dressing. The results were 16 g/100cm2.

## Example 4

The first and the second fabric in this example is silver nylon (PA) fabric, the middle layer is a chemically modified cellulose fabric.

The detailed manufacturing steps are:

A) The first and second layer use knitted silver nylon fabric, weight 31 gsm, volume density 0.14 g/cm3, grey in colour, silver content 22%.

The middle layer contains in-house made carboxymethyl cellulose fibres, linear density 2.2 dtex, fibre length 50 mm, the fibre's absorbency to Solution A is 2000%;

B) Take 500 g of CMC fibres, open and feed the fibres into a carding machine to make into a fibrous web;

C) Process the web into a needle punched felt as the middle layer fabric, needling density 40/cm2, fabric weight 125 gsm, volume density 0.075 gsm;

D) Place the above CMC felt between the first and the second fabrics, then feed them together into 2 needle machines respectively. The first machine needles downwards, the second upwards, needling density 100/cm2. This made the laminated three-layered fabric;

E) Cut the above laminated fabric into 10x10 cm, then pack and sterilize at a dosage of 25-50 KGy;

Test the absorbency of the finished dressing using the YY/T 047.1-2004 and EN 13726-1:2002/AC:2003 Test Method for Primary Wound Dressing. The results were 16 g/100cm2.

## Example 5

The first and the second fabric in this example is silver nylon (PA) fabric, the middle layer is a chemically modified cellulose fabric. The lamination method is ultrasonic welding.

The detailed manufacturing steps are:

F) The first and second layer use knitted silver nylon fabric, weight 129 gsm, volume density 0.32 g/cm3, black in colour, silver content 25%.

The middle layer contains in-house made carboxymethyl cellulose fibres and the PE/PP di-component fibres (ES-C). The linear density of the CMC fibre is 2.2 dtex, fibre length 50 mm, the fibre's absorbency to Solution A is 2000%; The linear density of the PE/PP fibre is 2.5 dtex, fibre length 45 mm

G) Take 500 g of CMC fibres and 100 g of ES fibre, blend evenly, open and feed the fibres into a carding machine to make into a fibrous web;

H) Process the web into a needle punched felt as the middle layer fabric, needling density 80/cm2, fabric weight 139 gsm, volume density 0.103 gsm;
I) Place the above CMC felt between the first and the second fabrics, then feed them together into an ultrasonic welding machine to laminate the three layers together. The laminated fabric has a diamond welding pattern;
J) Cut the above laminated fabric into 10x10 cm, then pack and sterilize at a dosage of 25-50 KGy;

Test the absorbency of the finished dressing using the YY/T 047.1-2004 and EN 13726-1:2002/AC:2003 Test Method for Primary Wound Dressing. The results were 15 g/100cm2.

In order to assess the dressing's antimicrobial function, the samples from the above example was cut into a 2x2 cm piece, then placed onto a Petri-dish that has been covered with Pseudomonas aeruginosa. The Petri-dish was then placed into an oven at a temperature of 37 °C for 24 hrs, and the growth of the bacteria around the dressing sample on the plate was observed (zone of inhibition). This demonstrates the dressing's ability to kill bacteria after 24 hrs contact time.

## Example 6

The first and the second fabric in this example is asymmetric PP fabric, the middle layer is Mid M alginate fabric (M:G equal ratio).

The detailed manufacturing steps are:

A) The first layer is a spun bond PP nonwoven, weight 12 gsm, volume density 0.08 g/cm3, white in colour. The layer is also a spun bond PP nonwoven, weight 34 gsm, volume density 0.11 g/cm3, orange in colour.
The middle layer contains in-house made MG alginate fibres, linear density 3.0 dtex, fibre length 75 mm, the fibre's absorbency to Solution A is 1400%;
The middle layer also contain PE/PP di-component fibres (ES-C) with a linear density of 2.5 dtex, fibre length 45 mm
B) Take 1000 g of alginate fibres, blended evenly with the 250 g ES-C fibre, then open and feed the fibres into a carding machine to make into a fibrous web;
C) Process the web into a needle punched felt as the middle layer fabric, needling density 60/cm2, fabric weight 365 gsm, volume density 0.065 gsm;
D) Place the above alginate felt between the first and the second fabrics, then place the fabrics together into an oven at 140 °C for 2 minutes, making the ES-C fibre melt and bind together. At the exit of the oven there is a pair of hot rollers (surface temperature 140 °C), the roller surface has a dot-pattern which further laminate the three layer and creates a dot pattern to the laminated fabric. The top of the fabric is white, bottom is orange.

E) Cut the above laminated fabric into 10x10 cm, then pack and sterilize at a dosage of 25-50 KGy;

Test the absorbency of the finished dressing using the YY/T 047.1-2004 and EN 13726-1:2002/AC:2003 Test Method for Primary Wound Dressing. The results were 14 g/100cm2.

The above examples illustrate the principle of the present invention, but do not limit the scope of this invention.

## Claims

1. A method of manufacturing a wound dressing, the wound dressing comprising: a first outer layer, a second outer layer, and a middle layer disposed between the first outer layer and the second outer layer; wherein the volume density of the first outer layer and a volume density of the second outer layer are greater than that of the middle layer;, wherein the method comprises the steps of: a) providing first and second outer layers, wherein the weights of the first outer layer and the second outer layer are between 8 and 140 grams per square meter (gsm), and the volume densities of the first outer layer and the second outer layer are between 0.08 and 0.32 g/cm3; b) providing a middle layer comprising at least one of the following fibres: alginate, chitosan, cellulose, carboxymethyl chitosan, acylated chitosan, carboxymethyl cellulose, carboxyethyl cellulose, water insoluble cellulose alkyl sulfonate fibre, bi-component fibre, polyvinyl alcohol, polypropylene, polyester, polyamide, polyacrylonitrile, cross-linked acrylates copolymer super absorbent fibres, and wood pulp and the weight of the middle layer is between 60-600 gsm; the fibre linear density of the middle layer is between 0.5-10 dtex; and the fibre length thereof is between 3-150 mm; and c) bonding the layers together.

2. The method of claim 1, **characterized in that** the first outer layer and the second outer layer comprise at least one of the following fibres: alginate, chitosan, cellulose, carboxymethyl chitosan, acylated chitosan, carboxymethyl cellulose, carboxyethyl cellulose, water insoluble cellulose alkyl sulfonate, polyvinyl alcohol fibre, polypropylene, polyester, polyamide, or polyacrylonitrile.

3. The method of claim 2, **characterized in that** at least one of the first outer layer and the second outer layer comprises one antimicrobial agent, and the concentration of the antimicrobial agent is between 0.01%-25% by weight of the corresponding layer; the middle layer comprises the antimicrobial agent, the concentration of the antimicrobial agent is between 0.01%-20% by weight of the middle layer.

**4.** The method of claim 3, **characterized in that** the antimicrobial agent is silver.

**5.** The method of claim 2, **characterized in that** at least one of the first outer layer and the second outer layer is a knitted fabric, a woven fabric, or a nonwoven fabric; the middle layer is a nonwoven fabric; the knitted fabric and the woven fabric are made from staple fibre yarn or filament; a linear density of the staple fibre yarn or the filament is between 0.5-10 dtex.

**6.** The method of claim 1, **characterized in that** the nonwoven fabric is made by needle punching, hydroentanglement, thermal bonding, or chemical bonding.

**7.** The method of claim 2, **characterized in that** the first layer, the second layer and the middle layer are laminated together by needle punching or thermal bonding or chemical bonding or ultrasonic welding or stitch bonding; the thermal bonding, stitch bonding and ultrasonic welding are adapted to produce patterns comprising square, triangle, rectangle, diamond, circle and dot.

**Patentansprüche**

**1.** Verfahren zum Herstellen eines Wundverbands, umfassend: eine erste Außenlage, eine zweite Außenlage und eine Mittellage, die zwischen der ersten Außenlage und der zweiten Außenlage angeordnet ist; wobei die Volumendichte der ersten Außenlage und eine Volumendichte der zweiten Außenlage größer als diejenige der Mittellage sind; wobei das Verfahren folgende Schritte umfasst: a) Bereitstellen einer ersten und zweiten Außenlage, wobei das Gewicht der ersten Außenlage und der zweiten Außenlage zwischen 8 und 140 Gramm pro Quadratmeter (gsm) beträgt und die Volumendichte der ersten Außenlage und der zweiten Außenlage zwischen 0,08 und 0,32 g/cm$^3$ beträgt; b) Bereitstellen einer Mittellage, die wenigstens eine der folgenden Fasern umfasst: Alginat, Chitosan, Cellulose, Carboxymethylchitosan, acyliertes Chitosan, Carboxymethylcellulose, Carboxyethylcellulose, wasserunlösliche Cellulosealkylsulfonatfaser, Zweikomponentenfaser, Polyvinylalkohol, Polypropylen, Polyester, Polyamid, Polyacrylnitril, Superabsorberfasern aus vernetztem Acrylat-Copolymer und Holzzellstoff, und das Gewicht der Mittellage zwischen 60 und 600 gsm beträgt; die lineare Faserdichte der Mittellage zwischen 0,5 und 10 dtex beträgt; und die Faserlänge derselben zwischen 3 und 150 mm beträgt; und c) Binden der Lagen aneinander.

**2.** Verfahren nach Anspruch 1, **dadurch gekennzeichnet, dass** die erste Außenlage und die zweite Außenlage wenigstens eine der folgenden Fasern umfassen: Alginat, Chitosan, Cellulose, Carboxymethylchitosan, acyliertes Chitosan, Carboxymethylcellulose, Carboxyethylcellulose, wasserunlösliche Cellulosealkylsulfonatfaser, Polyvinylalkoholfaser, Polypropylen, Polyester, Polyamid oder Polyacrylnitril.

**3.** Verfahren nach Anspruch 2, **dadurch gekennzeichnet, dass** wenigstens eine von der ersten Außenlage und der zweiten Außenlage ein antimikrobielles Mittel umfasst und die Konzentration des antimikrobiellen Mittels zwischen 0,01 Gew.-% und 25 Gew.-% der entsprechenden Lage beträgt; die Mittellage das antimikrobielle Mittel umfasst, wobei die Konzentration des antimikrobiellen Mittels zwischen 0,01 Gew.-% und 20 Gew.-% der Mittellage beträgt.

**4.** Verfahren nach Anspruch 3, **dadurch gekennzeichnet, dass** das antimikrobielle Mittel Silber ist.

**5.** Verfahren nach Anspruch 2, **dadurch gekennzeichnet, dass** wenigstens eine von der ersten Außenlage und der zweiten Außenlage ein Gestrick, ein Gewebe oder ein Vlies ist; die Mittellage ein Vlies ist; das Gestrick und das Gewebe aus Stapelfasergarn oder -filament hergestellt werden; eine lineare Dichte des Stapelfasergarns oder -filaments zwischen 0,5 und 10 dtex beträgt.

**6.** Verfahren nach Anspruch 1, **dadurch gekennzeichnet, dass** das Vlies durch Vernadeln, Hydroverfilzung, thermische Bindung oder chemische Bindung hergestellt wird.

**7.** Verfahren nach Anspruch 2, **dadurch gekennzeichnet, dass** die erste Lage, die zweite Lage und die Mittellage durch Vernadeln oder thermische Bindung oder chemische Bindung oder Ultraschallschweißen oder Stichverbinden aneinander laminiert werden; wobei die thermische Bindung, das Stichverbinden und das Ultraschallschweißen dazu angepasst werden, Muster zu erzeugen, die Quadrat, Dreieck, Rechteck, Raute, Kreis und Punkt umfassen.

**Revendications**

**1.** Procédé de fabrication d'un pansement, le pansement comprenant : une première couche extérieure, une deuxième couche extérieure et une couche centrale disposée entre la première couche extérieure et la deuxième couche extérieure ; dans lequel la densité volumique de la première couche extérieure et la densité volumique de la deuxième couche extérieure sont supérieures à celles de la couche centrale ; dans lequel le procédé comprend les étapes de : a) fourniture de première et deuxième couches extérieures, dans lequel les poids de la première couche extérieure et de la deuxième couche extérieure se situent entre 8 et 140 grammes par mètre carré (gsm), et les densités volumiques de la première couche extérieure et de la deuxième couche extérieure se situent entre 0,08 et 0,32 g/cm$^3$ ; b) fourniture d'une couche centrale comprenant au moins une des fibres suivantes : alginate, chitosane, cellulose, chitosane de carboxyméthyle, chitosane acylé, cellulose de carboxyméthyle, cellulose de carboxyéthyle, fibre de sulfonate d'alkyle de cellulose insoluble dans l'eau, fibre bi-composant, polyalcool de vinyle, polypropylène, polyester, polyamide, polyacrylonitrile, fibres super-absorbantes copolymères d'acrylates réticulés, et pâte de bois et dans lequel le poids de la couche centrale se situe entre 60-600 gsm ; la densité linéaire de fibre de la couche centrale se situe entre 0,5-10 dtex ; et la longueur de fibre de celle-ci se situe entre 3-150 mm ; et c) liaison des couches ensemble.

2. Procédé selon la revendication 1, **caractérisé en ce que** la première couche extérieure et la deuxième couche extérieure comprennent au moins une des fibres suivantes : alginate, chitosane, cellulose, chitosane de carboxyméthyle, chitosane acylé, cellulose de carboxyméthyle, cellulose de carboxyéthyle, sulfonate d'alkyle de cellulose insoluble dans l'eau, fibre de polyalcool de vinyle, polypropylène, polyester, polyamide, ou polyacrylonitrile.

3. Procédé selon la revendication 2, **caractérisé en ce qu'**au moins une de la première couche extérieure et de la deuxième couche extérieure comprend un agent antimicrobien, et la concentration de l'agent antimicrobien se situe entre 0,01 %-25 % en poids de la couche correspondante ; la couche centrale comprend l'agent antimicrobien, la concentration de l'agent antimicrobien se situe entre 0,01 %-20 % en poids de la couche centrale.

4. Procédé selon la revendication 3, **caractérisé en ce que** l'agent antimicrobien est de l'argent.

5. Procédé selon la revendication 2, **caractérisé en ce qu'**au moins une de la première couche extérieure et de la deuxième couche extérieure est un tissu tricoté, un tissu tissé, ou un tissu non tissé ; la couche centrale est un tissu non tissé ; le tissu tricoté et le tissu tissé sont fabriqués à partir d'un filament ou fil à fibres discontinues ; la densité linéaire du filament ou fil à fibres discontinues se situe entre 0,5-10 dtex.

6. Procédé selon la revendication 1, **caractérisé en ce que** le tissu non tissé est fabriqué par aiguilletage, hydroliage, liaison thermique ou liaison chimique.

7. Procédé selon la revendication 2, **caractérisé en ce que** la première couche, la deuxième couche et la couche centrale sont stratifiées ensemble par aiguilletage ou liaison thermique ou liaison chimique ou soudage par ultrasons ou liaison par point ; la liaison thermique, la liaison par point et le soudage par ultrasons sont adaptés pour produire des motifs comprenant carré, triangle, rectangle, losange, cercle et point.

Figure 1

Figure 2

1
2
3

Figure 3

Provide materials for all layers, such as the first layer fabric, the second layer and fibres of the middle layer. Take measurement of all parameters of the materials

Process the fibres into a web

Convert the web into a fabric for the middle layer

Laminate the third layer together with the first and the second layer fabric

Cut, pack or sterilise the dressing

Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0740554 A
- US 6471982 B
- US 6458460 B
- EP 0927013 A
- US 7385101 B
- EP 0575090 B1
- EP 0788378 B1
- WO 2010109239 A1
- CN 102481208 A
- US 20110280926 A1
- CN 102076291 A
- WO 2007117237 A1
- CN 101460202 A
- US 6552244 B
- EP 1139946 A
- CN 103126806 A
- GB 14335 T